# FASCICULE DE BREVET EUROPEEN

(11) **EP 0 702 951 B1**
(45) Date de publication et mention de la délivrance du brevet: **26.09.2001**
(21) Numéro de dépôt: 95401781.0
(22) Date de dépôt: 27.07.1995
(51) Int. Cl.: A61K 31/195, A61K 31/22

(54) **Utilisation d'un inhibiteur de la NO-synthétase comme médicament destiné à réduire la consommation d'alcool ou prévenir l'excès de consommation d'alcool**
Verwendung eines Inhibitors der NO-Synthetase als Medikament zur Reduktion des Alkoholkonsums oder zur Prävention eines exzessiven Alkoholkonsums
Use of a NO-synthetase inhibitor as medicament for reducing alcohol consumption or for preventing excessive alcohol consumption

(30) Priorité: 29.07.1994 FR 9409445
(43) Date de publication de la demande: 27.03.1996
(73) Titulaire: PERNOD-RICARD, 75008 Paris (FR)
(72) Inventeur: Beauge, Francoise, F-75013 Paris (FR)
(74) Mandataire: Warcoin, Jacques

(56) Documents cités:
- WO-A-93/05775
- ALCOHOLISM: CLINICAL AND EXPERIMENTAL RESEARCH (USA), 1995, VOL. 19, NO. 1, PAGE(S) 195-199, Adams M.L. et al 'Nitric oxide-related agents alter alcohol withdrawal in male rats'
- PHARMACOLOGY BIOCHEMISTRY AND BEHAVIOR (USA), 1995, VOL. 50, NO. 2, PAGE(S) 265-270, Rezvani A.H. et al 'Inhibition of nitric oxide synthesis attenuates alcohol consumption in two strains of alcohol-preferring rats'
- BRAIN RES BULL (UNITED STATES), 1993, VOL. 32, NO. 1, PAGE(S) 43-7, Khanna JM et al 'Inhibition of nitric oxide synthesis impairs rapid tolerance to ethanol.'
- EUR. J. PHARMACOL., vol.221, no.2-3, 1992 pages 399 - 400 Y.A. KOLESNIKOV ET AL. 'NG-Nitro-L-arginine prevents morphine tolerance'
- J HEPATOL (NETHERLANDS), MAR 1992, VOL. 14, NO. 2-3, PAGE(S) 146-50, HUNT NC ET AL 'Nitric oxide production by monocytes in alcoholic liver disease.'

## Description

La présente invention concerne un médicament destiné à prévenir l'excès de consommation d'alcool.

Le monoxyde d'azote NO est un messager rétrograde, produit par un certain nombre de neurones, impliqués dans les processus de mémorisation et d'adaptation, par exemple ceux de l'hypothalamus contenant de la vasopressine (Böhme et al. 1993).

Il a été étudié l'effet des inhibiteurs de NO-synthétase (NOS) sur l'hypotension de patients atteints de chocs septiques (Petros et al. 1991 et Lefroy et al. 1993). En particulier des inhibiteurs de NO-synthétase tels que le L-NMMA ou le L-NAME ont été administrés chez l'homme dans le traitement des manifestations hemodynamiques du choc septique ou de l'insuffisance hépatique.

Des études ont suggéré que la NO-synthétase pouvait jouer un rôle dans la prise de nourriture et la prise de poids subséquente (Morley et Flood 1994).

Il a enfin été montré que l'inhibition de la NO-synthétase diminue le phénomène dit de "tolérance rapide à l'alcool" (Khanna et al. 1993). Dans cette publication, les auteurs ont étudié l'adaptation à un test de coordination motrice lorsque la pratique de ce test se fait sous intoxication alcoolique forcée répétée. Dans ce cas, l'intoxication alcoolique proprement dite (donnée par l'importance des alcoolémies) n'est pas réduite. Les mêmes auteurs (Khanna et al. 1990) ont, par ailleurs, montré que le développement de la tolérance rapide ou aiguë n'était pas corrélé à la consommation d'alcool chez les mêmes animaux.

Aucune de ces publications n'a suggéré qu'un inhibiteur de NO-synthétase pouvait avoir un effet sur la consommation d'alcool.

On a découvert selon la présente invention qu'un inhibiteur de NO-synthétase a un effet sur le comportement de consommation d'alcool qu'il réduit, permettant de diminuer les effets délétères de l'alcool, notamment chez les sujets dépendants. Ainsi, la consommation d'alcool provoquée chez le rat, soit par "accès limité" (appétance), soit par "intoxication chronique par inhalation" (dépendance comportementale) se trouve empêchée ou réduite.

Il est particulièrement intéressant d'observer qu'à des doses où l'inhibiteur de NO-synthétase n'a pas ou très peu d'effet sur la prise journalière totale de liquide et la prise journalière totale de nourriture, la consommation d'alcool est réduite, à la fois en terme de quantité absolue d'alcool et en terme de proportion alcool/eau. Ceci montre que les effets sur la consommation de liquide et de nourriture décrits dans la littérature, et l'effet sur la consommation d'alcool selon la présente invention, sont de nature différente.

La présente invention a donc pour objet l'utilisation d'un inhibiteur de la NO-synthétase cérébrale pour préparer un médicament destiné à réduire la consommation d'alcool ou prévenir l'excès de consommation d'alcool chez les malades présentant une dépendance comportementale à l'alcool.

Ce médicament peut en particulier apporter une aide thérapeutique, notamment pendant les cures de desintoxication alcoolique.

Trois types de NO-synthétase ont été décrits dans le cerveau jusqu'à présent : deux constitutives cytosolique et membranaire et une inductible.

Parmi les inhibiteurs de NO-synthétase on cite plus particulièrement les inhibiteurs du sous-type de NO-synthétase constitutive et membranaire lié au récepteur du glutamate de type N-méthyl-D-Aspartate (NMDA).

Les principaux inhibiteurs des différentes NO-synthétases constitutives sont des dérivés de la L-Arginine qui est le substrat de ces enzymes dont notamment les dérivés L-NMMA, L-NA et L-NAME dont les formules sont données ci-après.

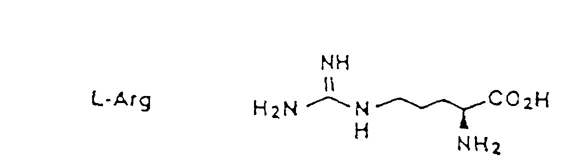

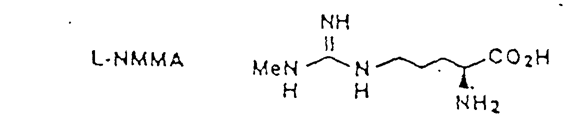

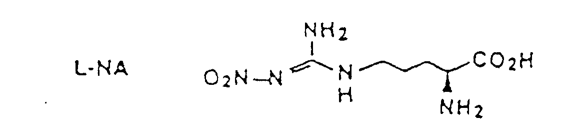

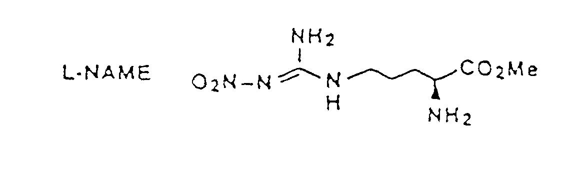

De préférence, on utilise la L-NA comme inhibiteur de NOS cérébrales selon la présente invention. D'autres avantages et caractéristiques de la présente invention apparaîtront à la lumière de la description détaillée qui suit.

Deux protocoles permettant une consommation volontaire significative d'alcool en libre choix (eau/alcool 10 %) chez le rat mâle et femelle ont été utilisés. Le premier protocole dit "d'accès limité" consiste à n'offrir ce libre choix que pendant 1 heure, chaque jour ; le second protocole consiste en un accès illimité au libre choix après éventuellement une période d'intoxication chronique importante par 'inhalation de vapeurs d'alcool (4 semaines) (protocole dif de "dépendance comportementale"). Dans les deux cas, quand la consommation d'alcool soit par heure, soit par jour s'est stabilisée, on administre une solution de L-nitroarginine (LNA), inhibiteur compétitif spécifique de la NO- synthétase cérébrale, i.p. à la dose de 50mg/kg de poids corporel, 30 minutes avant l'accès limité ou le soir avant la période nocturne en libre choix illimité. L'administration de L-NO-ARG fait baisser de 40 (chez les femelles) à 70 % (chez les mâles) la prise d'alcool moyenne dans les deux protocoles. Cette administration ne produit qu'une baisse légère et transitoire de la prise totale de liquide et de la prise de nourriture. La consommation d'alcool après injection de L-NO-ARG est réduite à la fois en terme de quantité absolue d'alcool et en terme de proportion alcool/eau (préférence). La coadministration de L-arginine et L-NO-ARG montre la spécificité de l'effet réducteur de la consommation d'alcool. Ces résultats montrent le rôle du NO dans la régulation de la prise d'alcool et la possibilité d'utiliser des inhibiteurs de la NO-synthétase dans le traitement de l'alcoolisme.

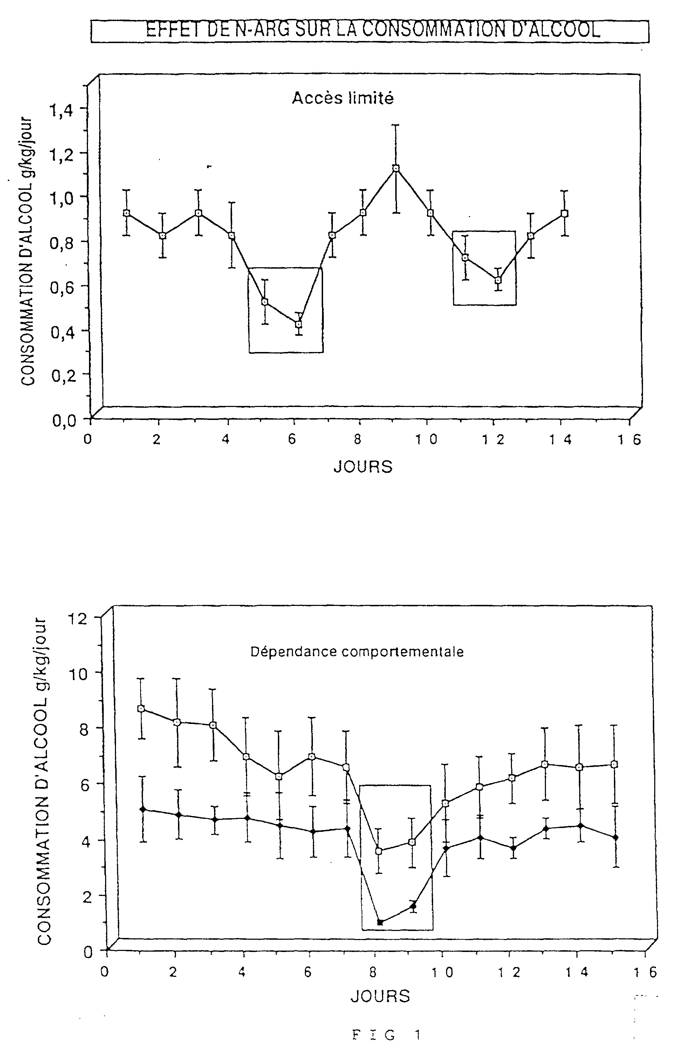

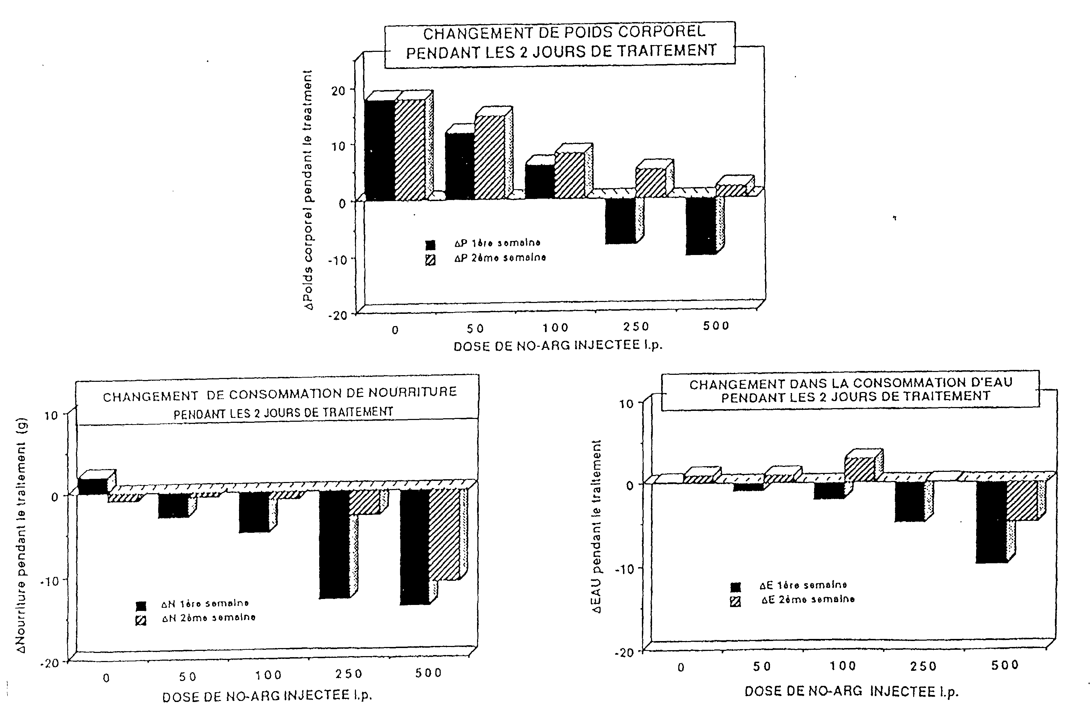

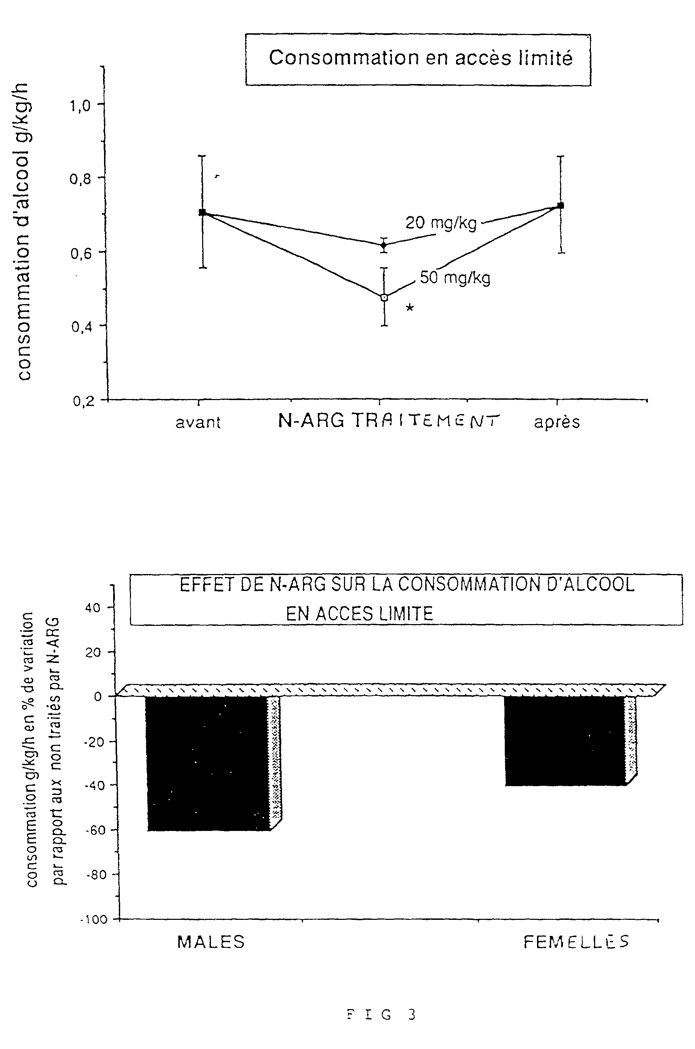

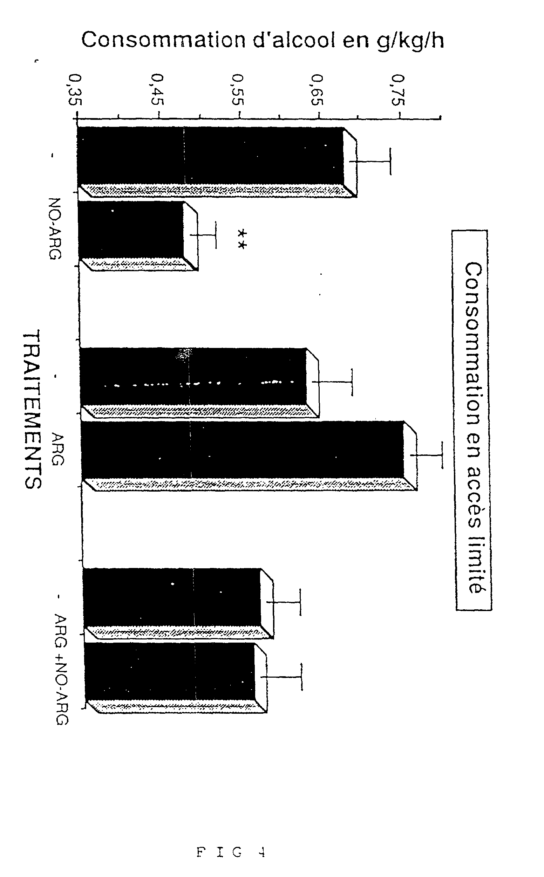

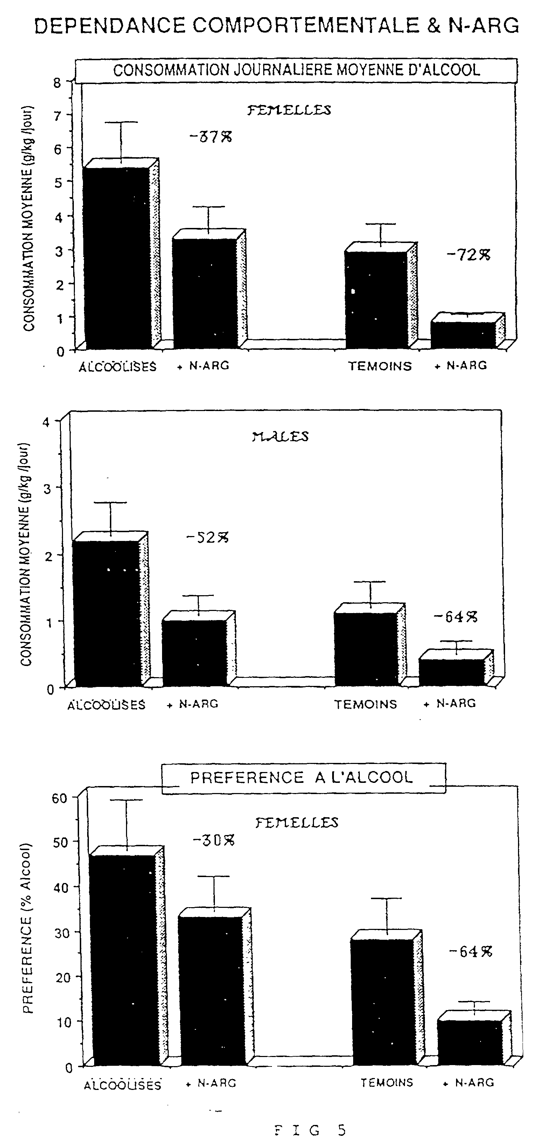

Les résultats sont présentés dans les figures 1 à 5.
. La figure 1 représente l'effet de la L-Nitro-Arginine sur la consommation d'alcool selon deux protocoles distincts. Dans les deux protocoles -accès limité et dépendance comportementale- les injections i.p. de L-NO-ARG ont été faites soit avant l'accès limité d'une heure, soit à 18h avant la période nocturne, deux jours consécutifs ou à une semaine de distance ; souvent les valeurs obtenues sur ces deux jours ont été moyennées.
. La figure 2 présente le changement de poids, de consommation de nourriture et de consommation d'eau pendant les deux jours de traitement. L'effet éventuellement toxique de ce protocole a été étudié par les variations de poids corporel, de consommation de nourriture et de consommation de liquide (eau) dans les mêmes conditions à plusieurs doses de L-NO-ARG : 50, 100, 250 et 500 mg/kg. On voit apparaître des diminutions significatives des trois paramètres uniquement à partir de 100mg/kg et on note à toutes doses une amélioration dans la 2ème semaine.
. La figure 3 présente l'effet dose dépendant de L-Nitro-Arginine sur la consommation d'alcool dans le protocole d'accès limité. On a observé lors du protocole d'accès limité que la dose nécessaire pour obtenir une diminution significative de consommation d'alcool par injection i.p. était de 50mg/kg et on s'en est tenu à cette dose dans les expériences suivantes : A 20mg/kg i.p., on observe un léger effet mais statistiquement non significatif. En revanche à 50mg i.p., le même effet devient nettement significatif. En général, les rats mâles répondent mieux que les rats femelles alors qu'ils consomment moins d'alcool.
. La figure 4 présente les effets comparatifs de L-Nitro-Arginine seule ou coadministrée avec la L-Arginine. Dans le protocole d'accès limité, on a testé la spécificité de l'action de L-NO-ARG. La L-ARG injectée à plus de 300mg/kg augmente très significativement la consommation de liquide mais a peut d'effet sur le niveau de consommation d'alcool ; coadministrée en excès à la dose de 100mg/kg avec la L-NO-ARG (50mg/kg), elle lève totalement l'inhibition de consommation d'alcool dûe à cet inhibiteur. Ce résultat reflète la compétition pour le site de liaison de L-ARG et démontre que la production de NO est impliquée dans le contrôle du comportement de consommation d'alcool.
. La figure 5 présente la consommation journalière moyenne d'alcool dans le protocole de dépendance comportementale. La situation est très semblable dans le protocole de dépendance comportementale. Il est à noter que les mâles et les animaux n'ayant pas subi d'intoxication chronique par inhalation avant le libre choix répondent plus. Enfin, les consommations d'eau importantes sur 24 heures nous ont permis de calculer un rapport de préférence solution alcoolisée/eau. La préférence pour l'alcool, compte tenu des faibles différences de consommation d'eau est fortement diminuée chez les animaux traités par L-NO-ARG.

### BIBLIOGRAPHIE

1. J.E. Morley & J.F. Flood ;
   Evidence that nitric oxide modulates food intake in mice. *Life Sciences* (1991) 49 : 707-711.
2. G. Calapai, F. Squadrito, D. Altavilla, B. Zingarelli, G.M. Campo, M.
   Cilia & A.P. Caputi ;
   Evidence that nitric oxide modulates drinking behaviour.
   *Neuropharmacology* (1992) 31 : 761-764.
3. L.E. Lambert, J.P. WHitten, B.M. Baron, H.C. Cheng, N.S. Doherty & I.A. Mc Donald ;
   Nitric oxide synthesis in the CNS, endothelium and macrophages differs in its sensitivity to inhibition by arginine analogues.
   *Life Sciences* (1991) 48 : 69-75.
4. M.A. Dwyer, D.S. Bredt & S.H. Snyder ;
   Nitric oxide synthase : irreversible inhibition by L-N^{G}-Nitroarginine in brain in vitro and in vivo.
   *Biochem*. *Biophys. Res*. *Comm.* (1991) 176 : 1136-1141.
5. C. Hölscher & S. P.R. Rose ;
   An inhibitor of nitric oxide synthesis prevents memory formation in the chick.
   *Neurosc*. *Letters* (1992) 145 : 165-167.
6. J.M. Khanna, G.S. Morato, G. Shah, A. Chau & H. Kalant ;
   Inhibition of nitric oxide synthesis impairs rapid tolérance to ethanol.
   *Braie Res. Bull*. (1993) 32 : 43-47.
7. Y. A. Kolesnikov, C. G. Pick & G. W. Pasternak ;
   N^{G}-Nitro-L-Arginine prevents morphine tolerance.
   *Eur*. *J*. *Pharmacol*. (1992) 221 : 399-400.
8. G. A. Böhme, C. Bon, M. Lemaire, M. Reibaud, O. Piot, J.M. Stutzmann,
   A. Doble & J.C. Blanchard,
   Altered synaptic plasticity and memory formation in nitric oxide synthase inhibitor-treated rats.
   *Proc*. *Natl*. *Acad*. *Sci*.,, *USA* (1993) 90 9191-9194.
9. J.E. Morley & J.F. Flood
   Effect of competitive antagonist of NO synthase on weight and food intake in obese and diabetic mice.
   *Am*. *J*. *Physiol*. (1994) 266 : R164-R168.
10. P. Klatt, K. Schmidt, F. Brunner & B. Mayer
   Inhibitors of brain nitric oxide synthase.
   *J*. *Biol*. *Chem.* (1994) 269 : 1674-1680.
11. A. Petros, D. Bennett, P. Vallance
   Effect of nitric oxide synthase inhibitors on hypotension in patients with septic shock.
   *Lancet* (1991) 338: 1557-1558.
12. D.C. Lefroy, D. Tousoulis & T. Crake
   Inhibition of nitric oxide synthesis.
   *Lancet* (1993) 342 : 1487-1488.
13. J.M. Khanna, H. Kalant, A.K. Chau & H. Sharma
   Initial sensitivity, acute tolerance and alcohol consumption in four inbred strains of rats.
   *Psychopharmacology* (1990) 101 : 390-395.

## Revendications

1. Utilisation d'un inhibiteur de la NO-synthétase cérébrale pour préparer un médicament destiné à réduire la consommation d'alcool ou à prévenir l'excès de consommation d'alcool chez les malades présentant une dépendance comportementale à l'alcool.

2. Utilisation selon la revendication 1, **caractérisée en ce que** l'inhibiteur de la NO-synthétase est un inhibiteur de NO-synthétase constitutive et membranaire lié au récepteur NMDA.

3. Utilisation d'un inhibiteur de la NO-synthétase cérébrale selon la revendication 2, **caractérisée en ce que** l'inhibiteur de la NO-synthétase est choisi parmi les dérivés de L-Arginine.

4. Utilisation selon la revendication 3, **caractérisée en ce que** l'inhibiteur de NO-synthétase est choisi parmi la L-NA, L-NMMA et L-NAME.

5. Utilisation selon la revendication 4, **caractérisée en ce que** l'inhibiteur de NO-synthétase est la L-NA.

6. Utilisation selon l'une quelconque des revendications 1 à 5, **caractérisée en ce que** ledit médicament est une composition dans laquelle ledit inhibiteur de la NO-synthétase cérébrale est présent à une dose inférieure à 100 mg/kg où la prise totale journalière de liquide et de nourriture n'est pas significativement réduite.

## Patentansprüche

1. Verwendung eines Inhibitors der zerebralen NO-Synthetase zur Herstellung eines Medikaments, das dazu bestimmt ist, den Alkoholkonsum zu verringern oder der Unmäßigkeit des Alkoholgenusses bei Kranken vorzubeugen, die eine verhaltensmäßige Abhängigkeit von Alkohol zeigen.

2. Verwendung nach Anspruch 1, **dadurch gekennzeichnet, dass** der Inhibitor der NO-Synthetase ein Inhibitor der NO-Synthetase ist, die als Bestandteil vom und membrangebunden am NMDA-Rezeptor vorliegt.

3. Verwendung eines Inhibitors der zerebralen NO-Synthetase nach Anspruch 2, **dadurch gekennzeichnet, dass** der Inhibitor der NO-Synthetase aus Derivaten von L-Arginin ausgewählt ist.

4. Verwendung nach Anspruch 3, **dadurch gekennzeichnet, dass** der Inhibitor der NO-Synthetase aus L-NA, L-NMMA und L-NAME ausgewählt ist.

5. Verwendung nach Anspruch 4, **dadurch gekennzeichnet, dass** der Inhibitor der NO-Synthetase L-NA ist.

6. Verwendung nach irgendeinem der Ansprüche 1 bis 5, **dadurch gekennzeichnet, dass** das Medikament eine Zusammensetzung ist, in welcher der Inhibitor der zerebralen NO-Synthetase in einer Dosis unterhalb von 100 mg/kg vorliegt, bei der die gesamte tägliche Aufnahme von Flüssigkeit und Nahrung nicht signifikant verringert ist.

## Claims

1. Use of a cerebral NO synthetase inhibitor for preparing a medicinal product intended to reduce the consumption of alcohol or to prevent excessive alcohol consumption in patients exhibiting a behavioural alcohol dependency.

2. Use according to Claim 1, **characterized in that** the NO synthetase inhibitor is a constitutive and membrane-bound NO synthetase inhibitor linked to the NMDA receptor.

3. Use of a cerebral NO synthetase inhibitor according to Claim 2, **characterized in that** the NO synthetase inhibitor is chosen from the derivatives of L-arginine.

4. Use according to the Claim 3, **characterized in that** the NO synthetase inhibitor is chosen from L-NA, L-NMMA and L-NAME.

5. Use according to Claim 4, **characterized in that** the NO synthetase inhibitor is L-NA.

6. Use according to any one of Claims 1 to 5, **characterized in that** the said medicinal product is a composition in which the said cerebral NO synthetase inhibitor is present at a dose of less than 100 mg/kg where the total daily intake of liquid and of food is not significantly reduced.
